# EUROPEAN PATENT APPLICATION

(11) **EP 1 894 547 A1**
(43) Date of publication of application: **05.03.2008**
(21) Application number: 06076639.1
(22) Date of filing: 29.08.2006
(51) Int. Cl.: A61F 5/448

(54) **Ostomy coupling**

(71) Applicant: Eurotec Beheer B.V., 4705 AG Roosendaal (NL)
(72) Inventor: Van der Leden, Arie Gijsbert, 2950 Kapellen (BE)

(57) **Abstract**

A low profile ostomy coupling system consisting of two circular coupling members (1,2) and a 'handcuff shaped' locking device (3). The first coupling member (1) consists of a polymeric flange (4) with an upstanding projecting rib (5), provided with a small ridge (6) alongside the outer circumference, and it is designed to be welded on the top layer of a skin friendly pressure sensitive adhesive (21) to be adhered to a patients skin. The second coupling member (2) consists of a radially outwards projecting 'U' shaped channel with uneven legs. The aperture of the second coupling member is sized to fit easily around the first coupling member, lightly jammed in between the flange and the small ridge (6) around its projecting rib. The channel is designed to fit the profile of the 'handcuff' locking device (3) and its largest leg is meant to be welded to a stoma appliance such as stoma pouch. The 'handcuff' locking device has a hinge (10) for easy positioning around the second coupling member, whereby its body profile fits exactly the channel of the coupling member (2). The ends of the 'handcuff' consist of a male (11) and a female (12) part, together forming a dual locking mechanism. The primary locking position keeps both arms of the handcuff together without pressure on the coupling members. The second locking position will be activated by putting more pressure on the male and female ends, thus squeezing the two coupling members together, which in practice will create a secure and leak proof connection between a stoma appliance and a body side wafer. To unlock, one simply pushes a button (15) and due to its spring in the hinge (16), the handcuff will unlock and slip back into its primary locking position.

## Description

### Background of the invention

A number of diseases involving the intestinal and urinary tracts may result in the construction of an artificial outlet on the abdominal area for stool and/or urine. In the case of diversion of the large intestine into the abdominal wall, one talks about a colostomy. In the case of diversion of the small intestine one talks about an ileostomy. Thirdly in the case of an urostomy, the urine is disposed through an artificial opening in the abdominal wall. The artificial openings of a colostomy, ileostomy and a urostomy are collectively referred to as stomas. During the past years a large variety of stoma appliances have been developed.

Patients with a colostomy collect the relatively normal faeces in closed pouches, which usually are changed several times a day. Patients with an ileostomy collect their relatively liquid stool in drainable pouches, the opening of which can be opened and closed with a tail clip. Patients with an urostomy collect their urine in a pouch with a drain tap. Ileostomy and urostomy pouches are usually changed once a day.

Stoma appliances are to be divided in two main systems; the 1-piece and the 2-piece system.

In case of a 1-piece system, the body side wafer - which consists of a special adhesive - is integral with the pouch. This type of pouch is designed to be affixed directly to the skin, and has the advantage that the total surface of the adhesive is flat and flexible, which allows it to follow the contours of the skin. However, the major disadvantage of an 1-piece system is the fact, that the adhesive has to be removed from the skin with every pouch change, which may cause skin irritations. In case of a 2-piece stoma system, the body side adhesive and the pouch are two separate components. First one affixes a special wafer and flange on to the skin. This flange is usually made from plastic and has a circular shape with an upstanding or projecting rib as a coupling member. The stoma pouch is also provided with a circular shaped coupling member, which can be attached on or around the projecting rib of the body side flange. A major advantage of a 2-piece stoma system is that the peristomal skin will not be disturbed during pouch changes, as the adhesive part will stay in place. A major disadvantage of the existing 2-piece stoma systems is that the body side adhesive wafer becomes less flexible, because of the rigidity and the higher profile of the in-built coupling member, which makes it less suitable for stomas positioned in difficult areas such as folds and irregularities of the skin. With both 1-piece and 2-piece stoma systems problems may occur in the post-operative period, when the wound is still fresh and the peristomal skin is very sensitive for pushing, pulling and sliding forces. In case of a 2-piece system an extra problem may occur, caused by a weak abdominal musculature, whereby the muscles are not able to give enough resistance, so that the attachment of the pouch on to the body side flange needs too much pressure, which may cause discomfort. The first widely used 2-piece stoma system designed by Steer et al (British patent application 571657). This stoma system consists of a body side wafer produced from a special hydrocolloid adhesive, covered by a thin polyethylene film, on which a circular shaped polymeric flange with an upstanding or projecting rib has been affixed, which functions as a coupling member. On to this coupling member a stoma pouch can be attached, which channel shaped coupling member is sized to snap securely over the projecting rib on the body side flange. This way of attaching a 2-piece stoma pouch may cause pressure on the sensitive skin, which - as mentioned before - can be very undesirable. Therefore the prior art has sought appliances which allow both the attachment and removal of pouches without burdening the sensitive or weak peristomal skin. In order to reduce the problems with the pressure forces when attaching a pouch of a 2-piece system on a sensitive or weak abdomen Holterman and

Hamelin developed a coupling system whereby the pouch flange can be attached on the body side wafer without pressure and secured by a rotating lock ring. (US patent 4929245). Later Holterman improved this system (US patent 5180377) by changing the locking system. With both systems the user locks the coupling system by pulling a handle.

A similar type of locking system on the body side wafer was invented by Steer (US patent 5843053). This systems functions opposite compared with that of Holterman, as the springy lock ring has to be unlocked when attaching or changing the pouch. The invention of Olsen (US patent 5496297) describes a locking system which is not part of the body side wafer, but hereby the locking ring is constructed within the flange system of each individual pouch.

The present invention relates also to an ostomy coupling of this kind, whereby the locking device is part of the pouch flange. However in this case the locking device is not a ring but a 'handcuff' like device, which the patients place around the pouch flange prior to attaching it to the body side wafer. After attaching it around the pouch flange in its primary locking position the pouch can smoothly be affixed to the body side flange. The second locking position will be activated by putting more pressure on the ends of the 'handcuff', thus squeezing the two coupling members together, which in practice will create a secure and leak proof connection between a stoma appliance and a body side wafer. To unlock, one simply pushes a button and due to its spring in the hinge, the handcuff will unlock and slip back into its primary locking position. The handcuff can be removed and be re-used again and again around the flanges of fresh pouches. So the 'handcuff' locking device of this invention in principal is a separate semi-disposable part of the ostomy system.

### Description of the invention

This invention concerns a low profile ostomy coupling system consisting of two circular coupling members and a 'handcuff shaped' locking device. The first coupling member (1) - the smallest in diameter - consists of a polymeric flange (4) with an upstanding projecting rib (5), provided with a small ridge (6) alongside the outer circumference. On the top of the inner wall of the projecting rib (5) a deflectable seal (7) is projecting downwards in order to lock certain other ostomy accessories, which however is not of any importance here and is not essential with regards to this invention.

The flange surface (4) is designed to be welded on the top layer of a skin friendly pressure sensitive adhesive(21) preferably a hydrocolloid body side wafer (20) to be adhered to a patients skin.

The second coupling member (2) consists of a radial outwards projecting channel shaped flexible flange of a U-shape with uneven legs (8). The aperture of this coupling member is sized to fit easily around the first coupling member (1), lightly jammed in between the flange (4) and the small ridge (6) around its projecting rib (5). The channel (9) is designed to fit the profile of the 'handcuff" locking device (3) and its largest leg (8b) is meant to be welded to a stoma appliance such as stoma pouch. The 'handcuff' locking device (3) has a hinge (10) for easy positioning it around the second coupling member (2), whereby its body profile fits exactly the channel of this coupling member. The ends of the 'handcuff" consist of a male part (11) and a female part (12). The male part (11) fits in the female part (12) together forming a dual springy locking mechanism. In primary locking position the bottom (12a) of the female part (12) is caught by a small protuberance (13) in the top of the male part (11), keeping both arms of the handcuff together in a rest position without much pressure on the second coupling member (2). This will usually be done before the aperture of the second coupling member (2) is put around the projecting rib (5) of the first coupling member (1), in between the flange (4) and the small ridge (6). When the first coupling member (1) en the second coupling member(2) are fitted together, the second locking position can be achieved by pushing the male end (11) and female end (12) of the 'handcuff' locking device (3) totally together whereby the bottom of the female part (12) will hook in the springy free space (14) of the projecting male part (11), thus also squeezing the two coupling members (1 and 2) together, which in practice will create a secure and leak proof connection between a stoma appliance and a body side wafer. To unlock, one simply pushes the springy button (15) and due to the 'spring' in the hinge (16), the 'handcuff' locking device (3) will unlock and slip back into its primary locking position (13). In order to increase comfort and enable opening and closing of the locking mechanism both outer end of the male part (11) and female part (12) are thickened and rounded (17) so that one gets more grip between finger and thumb when pushing the parts together. The second coupling member (2) has a decentralized groove (18) in the bottom of its channel to fit the deflectable springy slope protruding end (19) of the 'handcuff' locking device (3). Although the soft and elastic material of the second coupling member (2) already has a springy effect of itself, the deflectable springy slope protruding end (19) of the 'handcuff' locking device (3) is meant to spread and dose the pressure when squeezing both the ends (11) and (12) of locking device (3) together. Both the deflectable springy slope protruding end (19) and the groove (18) in the bottom of the channel of the second coupling member (2) are positioned decentralized in order to avoid that the user is attaching the 'handcuff" locking device (3) in the wrong direction around the second coupling member (2). Doing this wrongly the locking device (3) can not be closed and the second coupling member (2) will not fit easily around the protruding rib (5) of the first coupling member (1).

### Description of the drawings

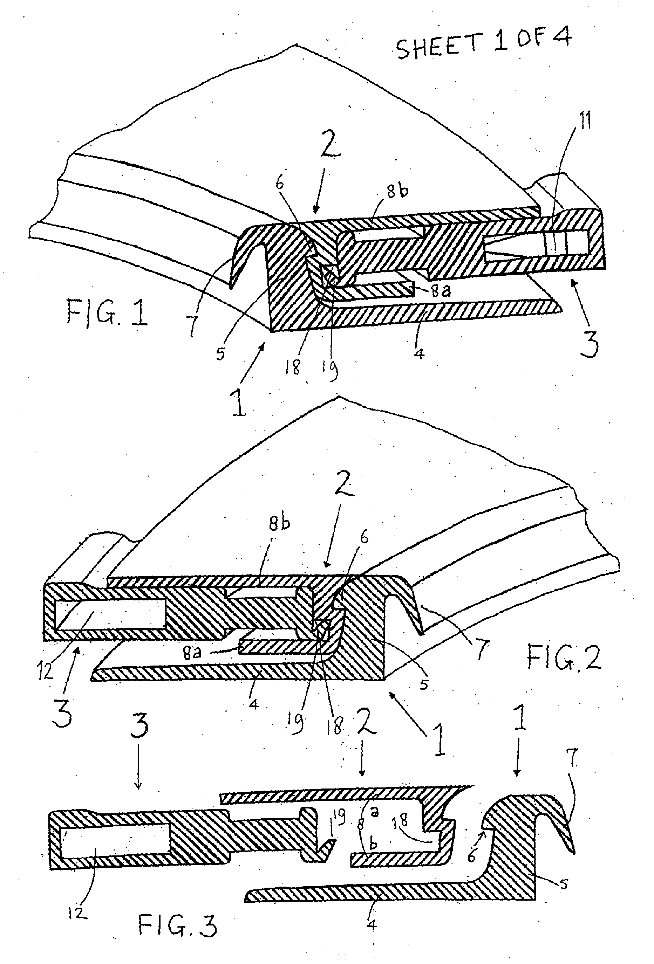

Figure 1 shows the 3 dimensional radial section through the tangent plane where the male and the female ends of the 'handcuff" locking device use to meet each other in second locking position (when the invention is totally closed), in this drawing seen in the direction of the male end of the 'handcuff' locking device.
Figure I shows the 3 dimensional radial section through the tangent plane where the male and the female ends of the 'handcuff' locking device use to meet each other in second locking position (when the invention is totally closed), in this drawing seen in the direction of the female end of the 'handcuff" locking device.
Figure 3 shows the radial section of the separated first coupling member, second coupling member and 'handcuff" locking device through the tangent plane of the female end of the 'handcuff" locking device.

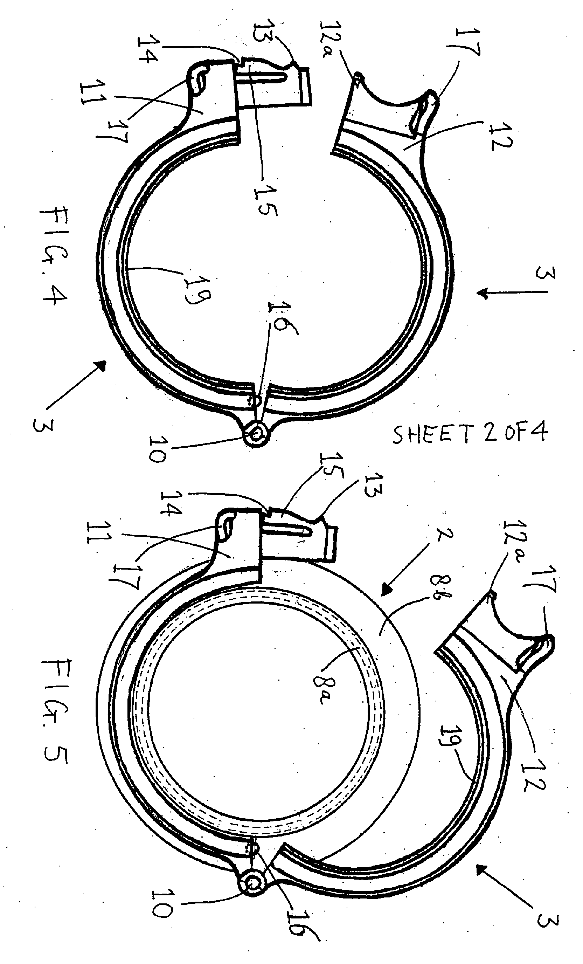

Figure 4 shows the 'handcuff' locking device in a totally open position.
Figure 5 shows the 'handcuff' locking device in a totally open position, whereby the lower half with the male end is already slid into the channel of the second coupling member.

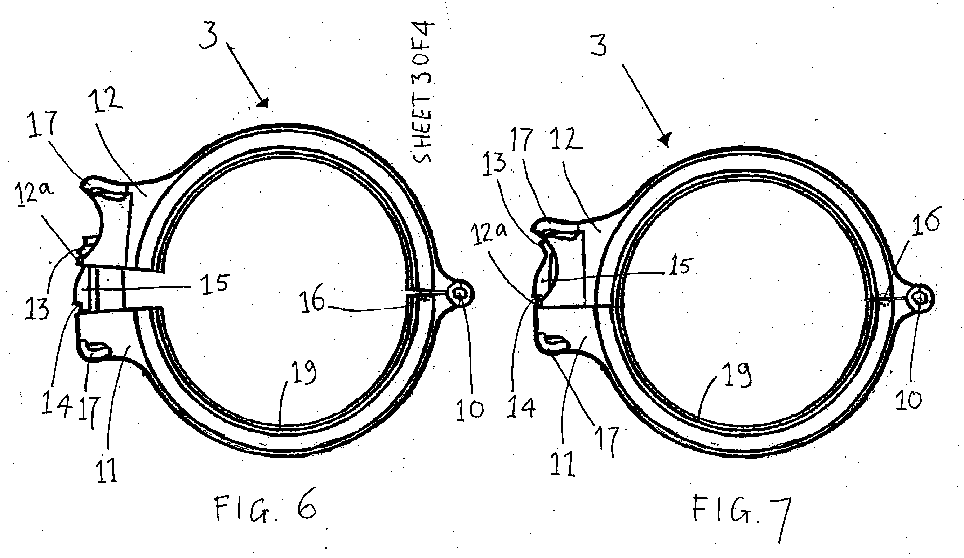

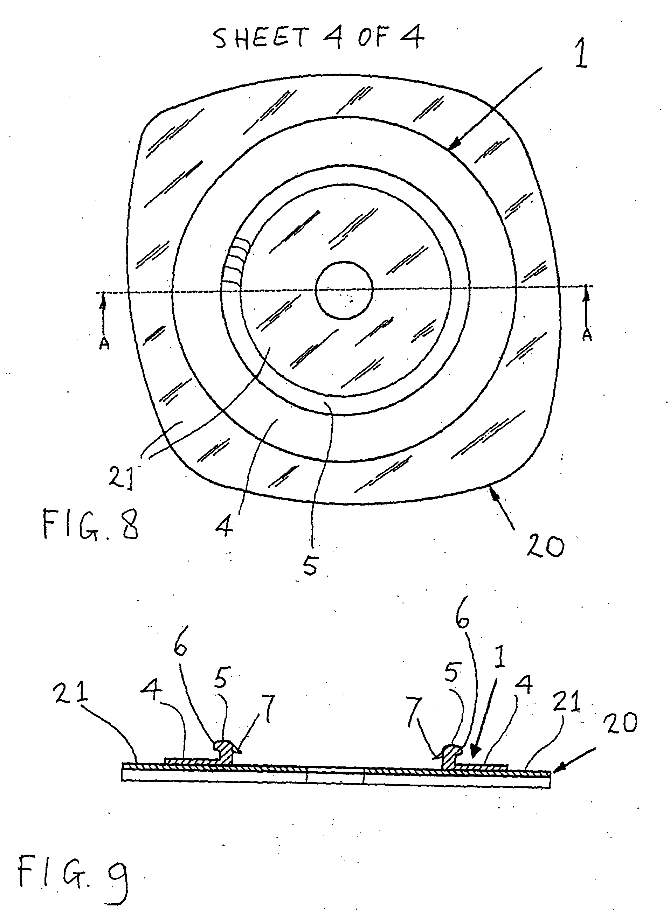

Figure 6 shows the 'handcuff' locking device in its primary locking position.
Figure 7 shows the 'handcuff' locking device in its secondary locking position (totally closed).
Figure 8 is a drawing, earlier used for US patent application 10/866,798 showing the first coupling member welded on the top layer a hydrocolloid body side wafer.
Figure 9 is a drawing, earlier used for US patent application 10/866,798 showing a cross section through the line marked 'A' in figure 8 of the first coupling member, welded on the top layer of a body side wafer.

## Claims

1. An ostomy coupling system **characterized by** the fact that it consists of
a) a first circular coupling member with a polymeric flange, designed to be welded on a body side wafer, which flange has an upstanding projecting rib provided with a small ridge alongside the outer circumference and
b) a second coupling member, designed to be welded on an ostomy pouch, which coupling member consists of a radial outwards projecting channel shaped flexible flange of a U-shape with uneven legs, whereby the aperture of this second coupling member is sized to fit easily around the first coupling member, lightly jammed in between the flange and the small ridge around its projecting rib and whereby its channel is designed to fit the profile of
c) a 'handcuff" locking device, whereby this 'handcuff' locking device has a hinge for easy positioning it in and around the channel of the second coupling member, and whereby the outer ends of this 'handcuff" locking device consist of a male part and a female part, which - when fitting into each other - together form a dual springy locking mechanism in two stages, whereby stage 1 is a primary locking position in which the bottom of the in the female part is caught by a small protuberance in the top of the male part, keeping both arms of the handcuff together in a rest position without much pressure on the second coupling member and whereby stage 2 is a second locking position, which can be achieved by pushing the male and female end totally together, whereby the bottom of the female part will be caught in the springy free space of the projecting male part thus also squeezing the two circular coupling members together, which in practice will create a secure and leak proof connection between the first and the second coupling member, which can be de-activated by pushing a radial inwardly moving button, so that the locking device will slip back from its second to its primary locking position.

2. An ostomy coupling system according to claim 1, whereby the second coupling member has a groove over the full circumference of the bottom of its channel and the 'handcuff' locking device has a rim with a deflectable springy slope protruding end over its full inner circumference, which rim fits exactly in said groove of the 'handcuff' locking device.

3. An ostomy coupling system according to claim 1, whereby the 'the second coupling member has a rim with a deflectable springy slope protruding end over the full circumference of the bottom of its channel and the 'handcuff" shaped locking device has a groove over its full inner circumference which groove fits exactly over said rim in the bottom of the channel of the second coupling member.

4. An ostomy coupling system according to claim 1, whereby the hinge of the 'handcuff' shaped locking device is not constructed as a conventional hinge with an axle, but simply consist of a flexible strip of plastic.

5. An ostomy coupling system according to claim 2, whereby the hinge of the 'handcuff' shaped locking device is not constructed as a conventional'hinge with an axle, but simply consist of a flexible strip of plastic.

6. An ostomy coupling system according to claim 3, whereby the hinge of the 'handcuff' shaped locking device is not constructed as a conventional hinge with an axle, but simply consist of a flexible strip of plastic.

7. An ostomy coupling system according to claim 2, whereby the male part and the female part of the 'handcuff' shaped locking device are constructed in opposite directions.

8. An ostomy coupling system according to claim 3, whereby the male part and the female part of the 'handcuff' shaped locking device are constructed in opposite directions.

9. An ostomy coupling system according to claim 5, whereby the male part and the female part of the 'handcuff' shaped locking device are constructed in opposite directions.

10. An ostomy coupling system according to claim 6, whereby the male part and the female part of the 'handcuff' shaped locking device are constructed in opposite directions.
